# EUROPEAN PATENT APPLICATION

(11) **EP 3 639 728 A1**
(43) Date of publication of application: **22.04.2020**
(21) Application number: 18794149.7
(22) Date of filing: 27.04.2018
(51) Int. Cl.: A61B 3/117

(54) **OPHTHALMOLOGIC DEVICE**

(30) Priority: 01.05.2017 JP 2017090979; 01.05.2017 JP 2017090980
(71) Applicant: Nidek Co., Ltd., Gamagori-shi, Aichi 443-0038 (JP)
(72) Inventor: HAMAGUCHI, Koji, Gamagori-shi Aichi 443-0038 (JP); BAN, Mitsuho, Gamagori-shi Aichi 443-0038 (JP)
(74) Representative: Zimmermann, Tankred Klaus
(86) International application number: PCT/JP2018/017310
(87) International publication number: WO 2018/203538

(57) **Abstract**

A controller 80 of an ophthalmic apparatus 1 acquires image data of an ACA image of an examinee's eye captured by a photographing optical system 30 which includes a light projecting optical system 40 for projecting light to an ACA region of the examinee's eye, and a light reception optical system 60 having a light reception element for receiving reflected light from the ACA region. Based on the image data, the controller 80 then causes the ACA image to be displayed on a monitor 8. At this time, the controller 80 further selects the ACA image display method between an upright display for displaying an upright image in which the top-bottom and right-left of the examinee's eye and the top-bottom and right-left of the ACA image on the monitor 8 correspond to each other, and a reverse display displaying a mirror image or an inverted image with respect to the upright image.

## Description

### TECHNICAL FIELD

The present invention relates to an ophthalmic apparatus for acquiring and displaying an anterior chamber angle image of an examinee's eye.

### BACKGROUND ART

For glaucoma diagnosis, it is useful to observe an anterior chamber angle (ACA) of the examinee's eye. Observation of the ACA has typically been performed visually by the examiner via a Gonio lens. As a type of Gonio lens, a reflection-type device is known. In the reflection type, the ACA is observed using a mirror image. As a type of Gonio lens, a refraction-type device is also known. In the refraction type, the ACA is observed using an upright image (or an inverted image).

With regard to the observation of the ACA, various apparatuses for photographing the ACA have been proposed in recent years (see Patent Literature 1, for example).

### CITATION LIST

### PATENT LITERATURE

PATENT LITERATURE 1: WO 2015/180923

### SUMMARY OF THE INVENTION

For example, in the case of observation of the ACA using a mirror image, it has been difficult for an inexperienced examiner to learn the correspondence between an observation image and an actual tissue in the examinee's eye.

In addition, in the Gonio lenses, there have long existed a reflection-type device, by which the ACA is observed using a reflection image, and a refraction-type device, by which an upright image (or an inverted image) is used for observation, for example. Accordingly, the idea of whether an upright image, a mirror image, or an inverted image should be preferable varies from one examiner to another.

An object of the present disclosure is to solve at least one of the problems of typical techniques and display an ACA image in a satisfactory manner.

An ophthalmic apparatus according to a first aspect of the present disclosure includes: an image data acquisition means which acquires image data of an ACA image of an examinee's eye captured by a photographing optical system which includes a light projecting optical system for projecting light to an ACA region of the examinee's eye and a light reception optical system having a light reception element for receiving reflected light from the ACA region; and a control means which causes the ACA image to be displayed on a monitor based on the image data, wherein the control means selects a display method for the ACA image between upright display for displaying an upright image in which the top-bottom and right-left of the examinee's eye correspond to the top-bottom and right-left of the ACA image on the monitor, and reverse display for displaying a mirror image or an inverted image with respect to the upright image.

An ophthalmic apparatus according to a second aspect of the present disclosure includes an image data acquisition means which acquires image data of an ACA image of an examinee's eye captured by a photographing optical system which includes a light projecting optical system for projecting light to an ACA region of the examinee's eye and a light reception optical system having a light reception element for receiving reflected light from the ACA region; and a control means which causes the ACA image to be displayed on a monitor based on the image data, and outputs, on the monitor, a position indicator indicating a position on the ACA region in association with all or a part of the ACA image.

According to the present disclosure, an ACA image can be displayed in a satisfactory manner.

### BRIEF DESCRIPTION OF THE DRAWINGS

Fig. 1 is a schematic diagram illustrating an overall configuration of an ophthalmic apparatus according to an Example.
Fig. 2 is a schematic diagram illustrating an example of a photographing optical system.
Fig. 3 is a block diagram of an example of a control system.
Fig. 4 illustrates an ACA image captured by the ophthalmic apparatus of the Example.
Fig. 5A illustrates an entire-circumference image using an upright image.
Fig. 5B illustrates an inverted image (180° reversed) with respect to Fig. 5A.
Fig. 5C illustrates a mirror image with respect to Fig. 5A.
Fig. 6 is a flowchart of an operation of the apparatus.
Fig. 7 is a flowchart of an alignment operation.
Fig. 8 illustrates a screen displayed during alignment.
Fig. 9 illustrates a listed display screen of photography results by a first layout.
Fig. 10 illustrates a listed display screen of photography results by a second layout, illustrating the case of reverse display.
Fig. 11 illustrates a listed display screen of photography results by the second layout, illustrating the case of upright display.
Fig. 12 illustrates a listed display screen of photography results by a third layout.

### DESCRIPTION OF THE EMBODIMENTS

### "Overview"

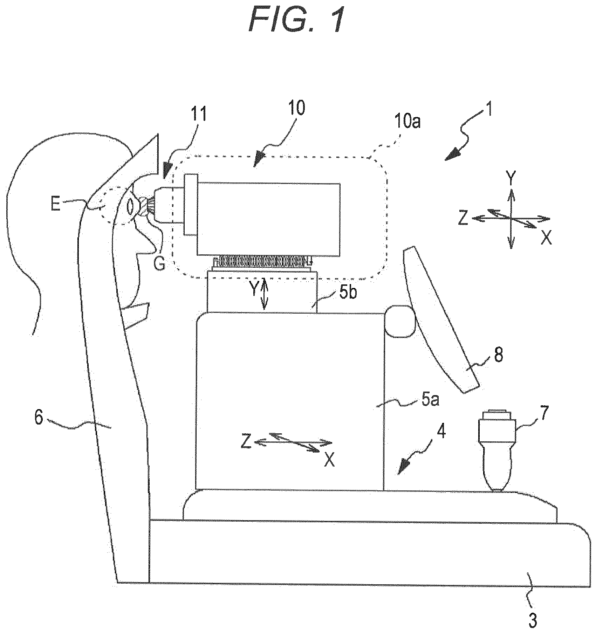

An ophthalmic apparatus of the present disclosure will be described with reference to an embodiment. The ophthalmic apparatus of the embodiment (see Fig. 1 to Fig. 3) may include at least an image data acquisition unit and a controller. Using the image data acquisition unit, the ophthalmic apparatus acquires image data of an ACA image of the examinee's eye photographed by means of a photographing optical system. Then, using the controller, the ophthalmic apparatus performs display control of the ACA image based on the image data.

### <Acquisition of image data of ACA image>

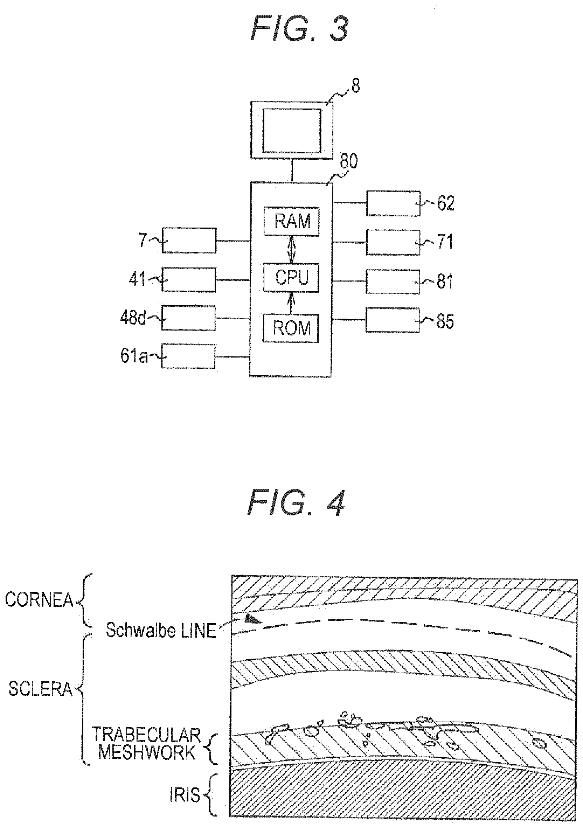

The ACA image may be a reflection image (see Fig. 4) formed (captured) based on reflected light from the ACA region of the examinee's eye. The ACA region may be a region including the ACA of the examinee's eye and a vicinity thereof.

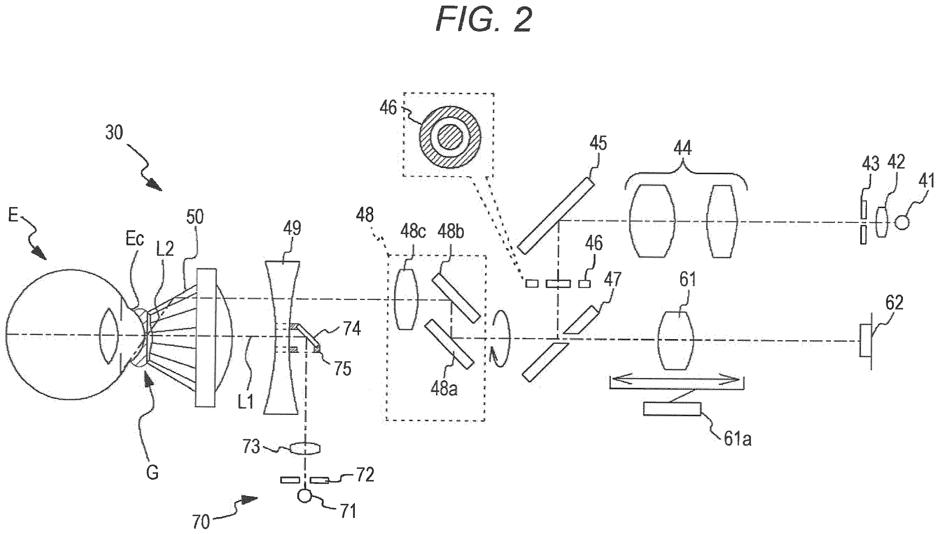

The ophthalmic apparatus may include a photographing optical system (see Fig. 2). In this case, the image data of the ACA image may be acquired as the result of photographing performed by the photographing optical system. For example, the image data is "acquired" as the image data is stored in a memory that a processor of the ophthalmic apparatus can access. Note that the image data of the ACA image may be image data of a still image, or image data of a moving image.

When the photographing optical system is included, the image data of the ACA image may be generated and acquired by a photography processing unit of the ophthalmic apparatus based on a light reception signal. As the photography processing unit, for example, a processor that governs the operation of the ophthalmic apparatus may be utilized, or a dedicated processing apparatus may be utilized.

### <Photographing optical system>

The photographing optical system (see Fig. 2) is utilized for photographing the ACA region of the examinee's eye. The photographing optical system performs projection of light to the ACA region along the photographing optical axis, and light reception of return light from the ACA region.

The photographing optical system may be configured to irradiate an entire photographing area of a surface tissue in the ACA with light, and to receive return light from the photographing area with a light reception element. In the following description of the embodiment, it is assumed that mainly, the photographing area of a single ACA image is a part of the entire circumference of the ACA. However, this is not a limitation, and the photographing area may cover the entire circumference of the ACA. Also, the photographing optical system may be able to switch the size of the photographing area, and may be able to capture ACA images of mutually different sizes of the photographing area. For example, the photographing optical system may be able to perform photographing by switching between an entire-circumference image in which the entire circumference of the ACA is captured in a single image, and a partial image in which an ACA region of a part of the entire circumference of the ACA is photographed. Note that the photographing area refers to a region of the ACA that is photographed (imaged) based on the input of a single photographing trigger, for example.

The light reception element may be a two-dimensional light reception element such as an imaging device. The two-dimensional light reception element captures, as the ACA image, an image of the ACA formed on a light reception surface. However, the photographing optical system is not necessarily limited to this. For example, the photographing optical system may be an optical system of line-scan or two-dimensional scanning type. In this case, the photographing area may be scanned (line scan or two-dimensional scan) with light, and the image data of the ACA image may be generated based on a light reception signal of return light for each scan. The image data of the ACA image may be that of an upright image in which the top-bottom and right-left of the image correspond to the top-bottom and right-left of the subject in the image, or a mirror image or an inverted image with respect to the upright image. That is, the image data may be that of an image which is reversed with respect to an upright image.

The photographing optical system includes at least a light projecting optical system and a light reception optical system. The light projecting optical system projects light to the ACA region of the examinee's eye. The light projected from the light projecting optical system may be visible light or invisible light (for example, infrared light). Also, the light may be monochromatic light or polychromatic light. The light reception optical system includes at least a light reception element that receives reflected light from the ACA region.

The photographing optical system may have a photographing optical axis which is inclined with respect to a fixation optical axis and directed toward the ACA region. Via the photographing optical axis, the light with respect to the ACA region is projected and received. The fixation optical axis is the projection optical axis of a fixation target with respect to the examinee's eye, for example. When the fixation target is projected, the ophthalmic apparatus may further include a fixation optical system for projecting the fixation target.

The photographing optical system may be provided with an objective optical system as follows. That is, the objective optical system may be provided which bends light from a light source so as to guide the light to the ACA region of the examinee's eye with the photographing optical axis inclined with respect to the fixation optical axis. The objective optical system in the photographing optical system may be a reflective system. That is, the objective optical system may include a member that reflects light, such as a mirror and a prism. The objective optical system may be disposed on the side closest to the examinee's eye in the photographing optical system. By the objective optical system, the light from the light source that is directed from the inside of the apparatus toward the outside is bent (for example, reflected) toward the fixation optical axis. In this way, the photographing optical axis inclined with respect to the fixation optical axis may be formed. Note that the objective optical system is not necessarily limited to a reflective system, and a part or all of the objective optical system may be formed from a refracting system (for example, a lens system).

### <Switching of ACA image photographing position>

When the ophthalmic apparatus includes the photographing optical system, the ophthalmic apparatus may further include a switch (which may also be referred to as a "photographing position switch") for displacing the photographing position of the photographing optical system with respect to the entire circumference of the ACA. The provision of the switch makes it possible to capture the ACA image in a plurality of photographing positions on the entire circumference of the ACA. As a result, the ophthalmic apparatus can acquire the image data of the ACA image captured in a plurality of photographing positions. At this time, as a type of photographing condition data indicating a photographing condition, data indicating the photographing position of each ACA image ("photographing position data") may be acquired (for example, stored in memory) in association with each ACA image.

Note that the ophthalmic apparatus may be an apparatus that captures the ACA image by selecting one of a plurality of photographing positions determined in advance, or an apparatus that captures the ACA image by setting a photographing position from the entire circumference of the ACA as desired.

When the photographing optical axis is inclined with respect to the fixation optical axis, the switch may be a unit that causes the photographing optical axis to be rotated around the fixation optical axis, for example. The switch may cause the photographing optical axis to be rotated around the fixation optical axis by causing a part or all of the photographing optical system to be rotated around the fixation optical axis. In this case, the switch may include a drive source such as a motor. Further in this case, data indicating the rotational position for each instance of photographing may be utilized as photographing position data indicating the photographing position.

Further, when the photographing optical system is an optical system of scanning type, an optical scanner for illuminating light scanning may be utilized as the switch. In this case, data indicating the drive position (i.e., scanning range) of the optical scanner may be utilized as the photographing position data.

Note that the photographing position data may be the result of detection of the drive position of the switch, or drive position command data, for example. When the photographing position data is acquired in terms of the result of detection of the drive position of the switch, a sensor for detecting the drive position (for example, a sensor for detecting the rotational position) may be included, and the photographing position data may be acquired based on a detection signal from the sensor.

Note that the switch is not necessarily limited to the above configuration. For example, instead of causing the photographing optical axis inclined with respect to the fixation optical axis to be rotated around the fixation optical axis, the switch may: be configured to guide fixation so that the orientation of the line of sight of the examinee's eye is displaced greatly: be configured to adjust the 3-dimensional positional relationship between the examinee's eye and the apparatus; or have a combination of the above configurations.

### <Display control of ACA image>

The controller (see Fig. 3) performs at least display processing of the ACA image. As the controller, a processor that governs the operation of the ophthalmic apparatus may be utilized, or a dedicated processing apparatus may be utilized.

In the present embodiment, as the result of display processing, one or a plurality of ACA images of the examinee's eye is displayed on a monitor. When a plurality of ACA images is simultaneously displayed on the monitor, the respective images may have mutually different photographing positions on the entire circumference of the ACA.

Further, the simultaneous displaying of a plurality of ACA images on the monitor may include displaying at least one composite image. A composite image is an image in which a plurality of ACA images is collaged.

The controller may form a composite image of a wide area with respect to a single image by having a plurality of ACA images with adjacent photographing positions collaged, and may cause the composite image to be displayed on the monitor. At this time, a plurality of ACA images corresponding to the entire circumference of the ACA may be collaged, for example. The collaged image corresponding to the entire circumference of the ACA may be generated as a linear image or an annular image. However, the collaged image does not necessarily need to correspond to the entire circumference, and a plurality of ACA images corresponding to a part of the entire circumference may be collaged, for example. In a concrete example, the entire circumference of the ACA may be divided into the four parts of nasal, superior, temporal, and inferior, and a collaged image may be generated for each part.

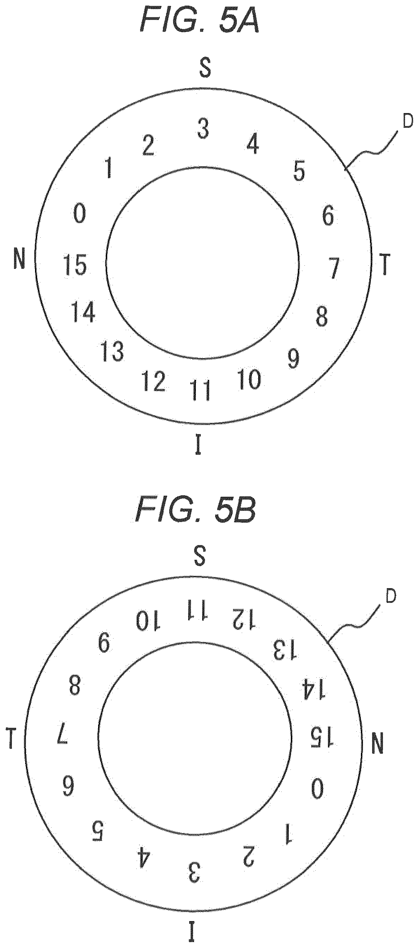

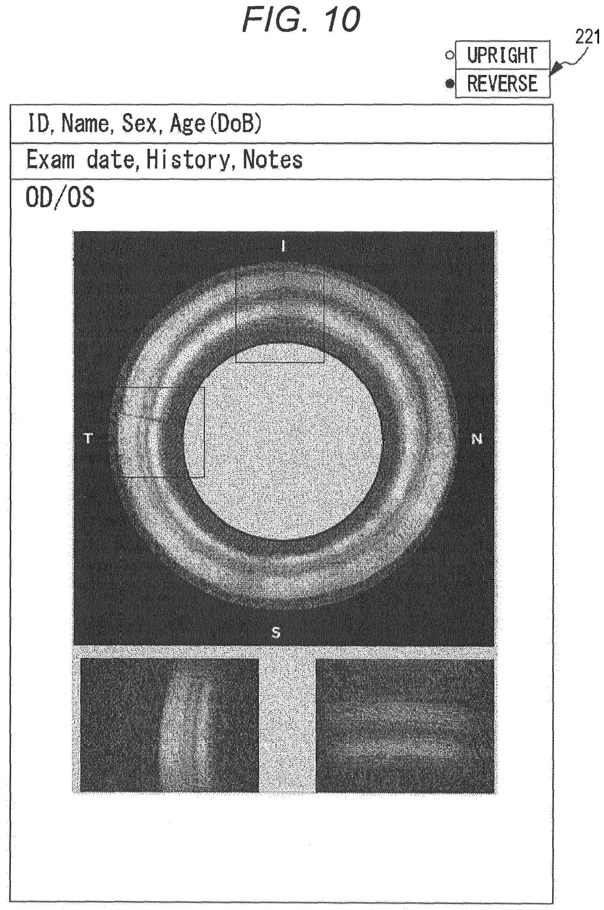

When a plurality of ACA images is displayed simultaneously on the monitor, the display position of each image may be associated with the photographing position on the entire circumference of the ACA in advance. At this time, the display position may be set in accordance with the positional relationship between the photographing positions. For example, the display position of the respective ACA images may be set such that the ACA images are disposed on the monitor in the positional relationship thereof when the examinee's eye is viewed from the front. Specifically, as illustrated in Fig. 5A and Fig. 10, a plurality of ACA images may be disposed in an annular manner on the monitor in the positional relationship thereof when the examinee's eye is viewed from the front.

### <Selection of display method>

In the present embodiment, the controller selects the ACA image display method between upright display and reverse display. When upright display has been selected, the ACA image is displayed as an upright image on the monitor. When reverse display has been selected, the ACA image is displayed as a mirror image or an inverted image with respect to the upright image on the monitor. In the upright image, the top-bottom and right-left of the ACA image on the monitor corresponds to the top-bottom and right-left of the examinee's eye. Also, the mirror image is captured by subjecting the upright image to mirror-image reversal with respect to one axis. The inverted image is captured by rotating the upright image by 180°.

The display method may be selected by the controller based on an operation input made by the examiner. In this case, it is possible to observe the ACA image by a display method desired by the examiner. That is, for an examiner skilled at visual ACA examination using the typical Gonio lens, it may be considered preferable to perform the observation using a reverse image. On the other hand, for an examiner inexperienced with ACA examination, it may be preferable to perform the observation using an upright image. As a consequence of the ability to select the display method based on an operation input made by the examiner, it becomes easier to perform the observation/diagnosis using an ACA image in a satisfactory manner.

Further, the ACA image display method may be selected by the controller automatically in accordance with the state of the apparatus. For example, the controller, when the examinee's eye and the apparatus are aligned, may cause the monitor to display the ACA image in the form of an upright image as an observation image for alignment. Because the direction of alignment error understood from the ACA image on the monitor and the direction of alignment error as viewed from the apparatus correspond to each other, it becomes easier for the examiner to learn from the ACA image on the monitor about in which direction an alignment error is present.

In the case where a plurality of ACA images is displayed at the display positions on the monitor corresponding to the respective photographing positions, the display position of the respective ACA images on the monitor may be switched in accordance with the selected display method.

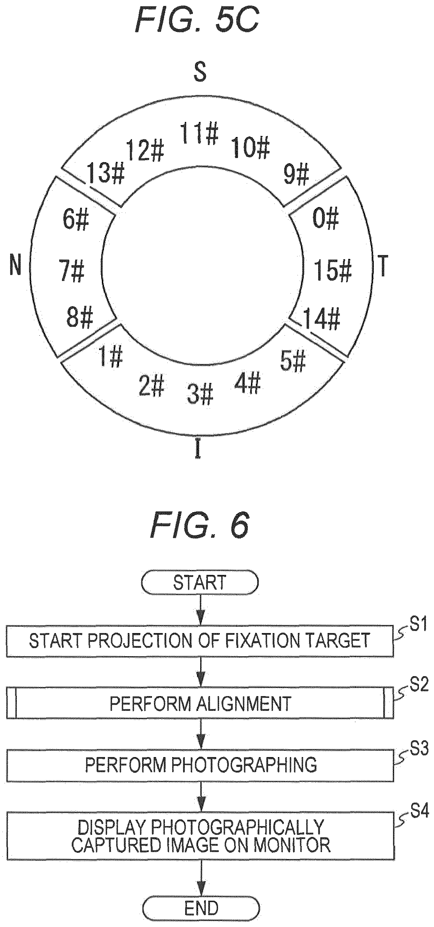

Referring to Fig. 5A to Fig. 5C, an example of the switching of the display position will be described. In Fig. 5A, a doughnut-shaped region D represents the entire-circumference image by upright image. Fig. 5B represents an inverted image (180°-reversed) with respect to Fig. 5A. Fig. 5C represents a mirror image with respect to Fig. 5A. Herein, the photographing positions in the entire-circumference image are indicated by the numbers "0" to "15". Note that in Fig. 5C, the "#" suffixed to each number indicates that a mirrored-reversal has been performed with respect to the upright image in each region.

As illustrated in Fig. 5A, when, for example, upright display has been selected as the display method, the correspondence between the display position of each ACA image on the monitor and each photographing position on the examinee's eye is matched in the top-bottom and right-left directions.

On the other hand, when reverse display has been selected as the display method, as illustrated in Fig. 5B and Fig. 5C, the correspondence between the display position of each ACA image on the monitor and each photographing position on the examinee's eye is reversed with respect to that in the case of upright display. At this time, with respect to the case of upright display, for example, the display positions may be exchanged between at least two ACA images of which the photographing positions on the entire circumference of the ACA are in a diagonal relationship (in other words, a symmetric relationship with respect to the visual axis).

For example, when upright display and inverted display are switched, the display position of each ACA image may be rotated by 180° on the screen together with the image (see Fig. 5B).

Also, when upright display and mirror-image display are switched, the display position of each ACA image may be subjected to mirror-image reversal with respect to one axis, together with the image.

Incidentally, in the typical reflection-type Gonio lens, a plurality (3 to 6) of reflecting surfaces is provided around the central axis. During observation via such Gonio lens, the entire circumference of the ACA is divided for each reflecting surface. That is, mirror images of regions divided in accordance with the number of the reflecting surfaces are observed. Accordingly, when the display position is switched between upright display and mirror-image display, for example, the controller may divide the entire circumference of the ACA into a plurality of parts (for example, the four parts of nasal, superior, temporal, and inferior), and the mirror-reversal and the change in display position may be performed on a part-by-part basis (see Fig. 5C).

Thus, as the display position of the respective ACA images on the monitor is switched in accordance with the selected display method, it becomes easier, whether upright display or reverse display is selected, for the examiner to learn the correspondence between each position on the entire circumference of the ACA and the ACA image in a satisfactory manner.

### <Position indicator>

The controller may output, to the monitor and together with the ACA image, a position indicator indicating the position on the ACA region in association with all or a part of the ACA image.

Information identifying the orientation of the ACA region being depicted in the ACA image may be output as the position indicator. The position indicator may indicate the absolute position of each photographing position, or the relative positions of photographing positions. Also, the position indicator may indicate a position determined in advance on the entire circumference of the ACA, and may include information indicating the image region corresponding to the position determined in advance.

The position indicator may include text information, such as letters, numbers, and signs, or graphical information such as figures. Of course, the position indicator may include information that indicates the photographing position by other methods.

The photographing position as the basis of the position indicator is identified based on, e.g., photographing condition data which corresponds to the ACA image and which is acquired in advance together with the ACA image. The controller outputs, to the monitor, the position indicator indicating the photographing position identified based on the photographing condition data. Using the position indicator indicating the position on the ACA region in association with all or a part of the ACA image, the examiner can easily associate the ACA image with the site of the examinee's eye that has been photographed.

When a plurality of ACA images is displayed on the monitor, the position indicator may be displayed with respect to all of the ACA images, or the position indicator may be displayed with respect to some of the ACA images. Also, the position indicator may be displayed for each of image regions included in a single ACA image.

For example, when the entire-circumference image, which is the ACA image representing the entire circumference of the ACA, and the partial image, which is the ACA image representing the region of a part of the entire circumference of the ACA, are displayed on the monitor simultaneously or in a switched manner, the controller may display the position indicator as follows. For example, a common position indicator may be displayed in association with each of a partial image and an image region corresponding to the partial image in the entire-circumference image. Also, an indicator that highlights an image region corresponding to a partial image in the entire-circumference image may be displayed as the position indicator in the partial image. By performing such display, it becomes possible for the examiner to easily learn the correspondence between the partial image and the region corresponding to the partial image in the entire-circumference image.

Note that the entire-circumference image may be a composite image in which a plurality of ACA images with mutually different photographing positions is collaged. In the case of a composite image, it may become difficult in some cases for the examiner to learn information about the peripheral part of a partial image as a result of its having been composed with another partial image. In this regard, by displaying not only the composite image but also the partial images, it becomes possible for the examiner to observe the peripheral part of each partial image in a satisfactory manner.

Further, if the display position of a plurality of ACA images being displayed on the monitor is changed by the switching of the display method between upright display and inverted display, it may become difficult for the examiner to learn the photographing position in each ACA image. However, in the present embodiment, the position indicator indicating the photographing position is displayed in association with all or a part of the ACA image. Accordingly, even if the display position of a plurality of ACA images on the monitor is changed, it is possible for the examiner to easily learn the photographing position in each ACA image. Also, the display position of the position indicator on the monitor may be switched in accordance with the display method that has been selected.

### <Alignment driver>

The ophthalmic apparatus may further include an alignment driver. The alignment driver modifies the positional relationship between the examinee's eye and the photographing optical system. In this case, alignment adjustment is performed by causing the examinee's eye and the photographing optical system to be moved relative to each other. The alignment driver may modify the positional relationship with regard to any of front-rear direction, right-left direction, and top-bottom direction.

For example, the alignment driver may be a unit that causes the photographing optical system to be moved relative to the examinee's eye. The alignment driver may include a mechanical mechanism, a mechanism that causes the photographing optical system to be moved by an electric actuator, or a mechanism including both. The alignment driver may be driven manually in accordance with the operation of an operating unit (for example, a joystick), or may be driven automatically based on the ACA image, for example.

### <Operation concerning alignment>

For example, the alignment adjustment for the examinee's eye and the photographing optical system may be performed utilizing a feature of the ACA. The following "Example" will be described mainly with reference to the trabecular meshwork as a feature. It should be noted, however, that tissues other than the trabecular meshwork may be utilized as a feature. As other features, it is possible to utilize the Schwalbe's line, scleral spur, ciliary zonule, iris, a feature due to lesion, and the like.

During the alignment utilizing the feature in the present embodiment, the controller performs photographing of the ACA, the positional relationship between the examinee's eye and the photographing optical system is adjusted based on the position of the feature included in the ACA image, and, as a result, the feature comes to be included in the photographing area in a satisfactory manner.

### "Example"

In the following, an Example of the ophthalmic apparatus according to the present disclosure will be described with reference to the drawings. The ophthalmic apparatus 1 of the Example is an ACA photographing apparatus. The ophthalmic apparatus 1 captures a reflection image (see Fig. 4) of the ACA of the examinee's eye.

### <Apparatus configuration>

Referring to Fig. 1, a schematic apparatus configuration of the ophthalmic apparatus 1 will be described. Note that the following description will be made assuming that, in Fig. 1, the X-direction corresponds to the right-left direction, the Y-direction to the top-bottom direction, and the Z-direction to the front-rear direction.

The ophthalmic apparatus 1 irradiates the examinee's eye E with illuminating light in a diagonal direction with respect to the visual axis. That is, the photographing optical axis in the ophthalmic apparatus 1 is inclined with respect to the fixation optical axis, and is directed toward the ACA region. The ophthalmic apparatus 1 receives reflected light from the ACA region along the photographing optical axis, and thereby captures a reflection image of the ACA region of the examinee's eye as the ACA image.

As illustrated in Fig. 1, the ophthalmic apparatus 1 includes a base 3, alignment mechanisms 4, 5a, 5b, a face support unit 6, a joystick 7, a monitor 8, an optical unit 10, and the like.

The optical unit 10 includes a major optical system utilized for capturing a reflection image of the ACA. The details of the optical system will be described later with reference to Fig. 3. In the Example, the optical unit 10 is accommodated in a cover 10a. However, a tip-end portion 11 is exposed outside the cover 10a.

The base 3 supports the alignment mechanisms 4, 5a, 5b and the face support unit 6.

In the Example, the alignment mechanisms 4, 5a, 5b generally include a moving base 4 and XYZ-drivers 5a, 5b. The moving base 4 of these is actuated by a mechanical mechanism, and the XYZ-drivers 5a, 5b are actuated by electric-type actuators.

The moving base 4 is disposed on the base 3, and includes a mechanical moving mechanism disposed between the moving base 4 and the base 3. The moving mechanism causes the moving base 4 to be moved in the X- and Z-directions, thereby adjusting the positional relationship between the examinee's eye E and the optical unit 10 concerning the X- and Z-directions. The examiner operates the joystick 7 to cause the moving base 4 to be moved relative to the base 3.

In the Example, the XYZ-drivers 5a, 5b are further mounted on the moving base 4. The XYZ-drivers 5a, 5b, based on control signals from the controller 80 (see Fig. 3) of the ophthalmic apparatus 1, cause the optical unit 10 to be moved in each of the X-, Y-, and Z-directions. As a result, with respect to each of the X-, Y-, and Z-directions, the positional relationship between the examinee's eye E and the optical unit 10 is adjusted.

The monitor 8 is disposed on the side of the housing on the examiner side. The monitor 8 may be a display that displays the ACA image captured via the optical unit 10.

### <Optical system>

Next, referring to Fig. 2, the optical system provided in the optical unit 10 will be described. The optical unit 10 includes at least a photographing optical system 30. Also, in the Example, the optical unit 10 further includes a fixation optical system 70.

### <Fixation optical system>

For convenience, the fixation optical system 70 will be described first. The fixation optical system 70 includes at least the fixation light source (fixation target) 71. Also, in Fig. 2, the fixation optical system 70 further includes an aperture 72, a lens 73, and a mirror 74. Light (fixation light flux) emitted from the light source 71 passes through the lens 73 via the aperture 72, whereby the light is collimated into a predetermined light flux size. The collimated light is bent by the mirror 74. The fixation light flux then passes through the center of a lens 49 and an objective reflector 50 (a prism in the Example) and is projected toward the examinee's eye E. Here, the mirror 74 is disposed in a position such that the illuminating light projected and received with respect to the ACA is not blocked in the photographing optical system 30. For example, the mirror 74 is disposed at the center of the lens 49.

In Fig. 2, the optical axis of the fixation optical system 70 (more specifically, the area of the optical axis of the fixation optical system 70 from the mirror 74 to the examinee's eye E) is represented by sign LI, and is referred to as a fixation optical axis. The members of the photographing optical system 30 illustrated in Fig. 2 are disposed with reference to the fixation optical axis L1.

### <Photographing optical system>

The photographing optical system 30 includes a light projecting optical system 40 and a light reception optical system 60. Also, the photographing optical system 30 includes a photographing optical axis L2. The photographing optical axis L2 is inclined with respect to the fixation optical axis L1, and is directed toward the ACA of the examinee's eye E.

The light projecting optical system 40 includes at least an objective reflector 50 and a light-decentering section 48. In the Example, the light projecting optical system 40 also includes a light source 41, a lens 42, an aperture 43, a lens 44, a mirror 45, a ring aperture 46, a perforated mirror 47, and a lens 49.

The light source 41 is a light source for the illuminating light with which the ACA is irradiated. In the Example, the light source 41 emits visible light. In the following description, in order to obtain the ACA image as a color image, it is assumed that at least a plurality of colors of light (for example, white light) having different wavelength regions can be emitted.

The light (illuminating light) from the light source 41 passes through the lens 42, the aperture 43, the lens 44, the mirror 45, the ring aperture 46, and the perforated mirror 47, and enters the light-decentering section 48.

Here, the ring aperture 46 is provided to suppress stray light due to reflections in the photographing optical system 30. For example, the ring aperture 46 suppresses reflections by the surfaces of the lens 48c, lens 49, and the like on the light source 41 side.

Further, the perforated mirror 47 is an example of an optical path splitter for splitting the optical path between the light projecting optical system 40 and the light reception optical system 60. Instead of the perforated mirror 47, a beam splitter such as a half-mirror may be applied. In the Example, the light from the light source 41 is reflected by a mirror surface of the perforated mirror 47 to travel toward the light-decentering section 48.

Note that in the Example, the optical-path center of the illuminating light reflected by the perforated mirror 47 is coaxial with the fixation optical axis L1.

The light-decentering section 48 causes the optical path of the illuminating light to be decentered with respect to the fixation optical axis L1. In the Example, two mirrors 48a, 48b which are disposed in parallel are used to cause the optical-path center of the illuminating light to be shifted by a predetermined interval with respect to the fixation optical axis L1. The shifted illuminating light passes through the lens 48c and is irradiated out of the light-decentering section 48.

The lens 49 and the objective reflector 50 have their respective optical axes disposed at positions spaced apart from the optical-path center of the illuminating light that has been decentered by the light-decentering section 48. In the Example, the respective optical axes of the lens 49 and the objective reflector 50 are disposed coaxially with the fixation optical axis L1.

The illuminating light passes through a peripheral portion (region spaced apart from the optical axis) of the lens 49 and is guided to the objective reflector 50. In the Example, the lens 49 is a lens with a minus power. The lens 49 causes the illuminating light emitted from the light-decentering section 49 substantially in parallel to the fixation optical axis L1 to be bent in an orientation away from the fixation optical axis L1 and to enter the objective reflector 50.

The objective reflector 50 has a reflecting surface for bending the illuminating light toward the fixation optical axis L1. The optical axis of the illuminating light reflected by the reflecting surface is bent so as to be inclined greatly with respect to the fixation optical axis LI, and guided to the outside of the apparatus. At this time, the optical axis that has been guided to the outside of the apparatus is utilized as the photographing optical axis L2 in the Example. The illuminating light from the apparatus travels along the photographing optical axis L2 and irradiates the ACA region of the examinee's eye E.

In the Example, the objective reflector 50 has a plurality of reflecting surfaces arranged around the optical axis. As a concrete example of the objective reflector 50, in the Example, a frustum-shaped prism having a regular polygon on the bottom face is utilized, for example. More specifically, for the bottom face, a prism with a regular hexadecagon shape with 16 sides is utilized. In the present embodiment, the reflecting surfaces directed toward the fixation optical axis L1 are disposed at the respective directions of 0°, 22.5°, 45°, 67.5°, 90°, ..., and 337.5° as viewed from the examinee's eye E. Note that the respective ACAs are ACAs with reference to the fixation optical axis L1. Also, for convenience of description, it is assumed that 0° is on a horizontal plane (more specifically, on the right side of the horizontal plane as viewed from the ophthalmic apparatus 1).

It should be noted, however, that the reflecting surface is not required to be divided into a plurality of surfaces, and may be formed of a continuous curved surface. The objective reflector 50 is also not required to be a prism, and may be a reflecting mirror. In this case, the reflecting mirror may be a tubular polygonal mirror or curved mirror with reflecting surfaces on the optical axis side.

Here, in the Example, the ophthalmic apparatus 1 includes a driver 48d (see Fig. 3) for causing the light-decentering section 48 to be rotated around the fixation optical axis L1. In accordance with the rotation of the light-decentering section 48, the entry position of the illuminating light with respect to the lens 49 and the objective reflector 50 is rotated around the fixation optical axis L1. As a result, the photographing optical axis L2 is rotated around the fixation optical axis LI, and, consequently, the illuminating light-irradiated position on the entire circumference of the ACA is displaced.

In the Example, a gel G is interposed between the objective reflector 50 (prism) and the cornea. The gel G is applied to the cornea, for example, to suppress corneal reflection of the illuminating light and to mitigate refraction at the tip-end interface of the objective reflector 50. Note that the gel G may be in a state of being filled in a holding container, not shown, and may be in contact with both the cornea and the tip-end of the objective reflector 50 (for more details, refer to "JP-A-2002-17680" by the present applicant, for example).

The illuminating light irradiated by the light projecting optical system 40 is reflected by the ACA region, and is guided to the light reception optical system 60 in the apparatus along the photographing optical axis L2.

In the Example, the light reception optical system 60 includes at least an imaging device (an example of light reception element) 62. The light reception optical system 60 also shares at least the objective reflector 50 and the perforated mirror (beam splitter) 47 with the light projecting optical system 40. The light reception optical system 60 may further share the light-decentering section 48, the lens 49, and the like with the light projecting optical system 40. The light reception optical system 60 also includes a focusing lens 61. The focusing lens 61 is a part of a focus-modifier of the photographing optical system 30 of the Example. The ophthalmic apparatus 1 is provided with a driver 61a for causing the focusing lens 61 to be moved along the optical axis. The driver 61a may include a linear-motion actuator, for example.

The reflected light from the ACA region irradiates the perforated mirror 47 via the objective reflector 50, the lens 49, and the light-decentering section 48. Thereafter, the reflected light passes through the opening of the perforated mirror 47 and the focusing lens 61, and forms an image at the imaging device 62. Consequently, based on a light reception signal from the imaging device 62, an ACA image with the photographing position corresponding to the site irradiated with the illuminating light on the entire circumference of the ACA is obtained. The ACA image obtained as a so-called raw image may be an upright image or a reverse image.

Further, by causing the light-decentering section 48 to rotate and causing the photographing optical axis L2 to be rotated around the fixation optical axis LI, it becomes possible to switch the photographing position on the entire circumference of the ACA. As discussed above, in the Example, the objective reflector 50 has 16 reflecting surfaces. Accordingly, it is possible to divide the entire circumference of the ACA into 16 portions and photograph each selectively.

### <Control system>

Next, referring to Fig. 3, the control system of the ophthalmic apparatus 1 will be described. The ophthalmic apparatus 1 is provided with a controller (processor) 80. The controller 80 performs a control process for the apparatus as a whole and various computing processes.

The controller 80 may include a CPU, a ROM, a RAM and the like. In the RAM, temporary data used for photographing and measurements are stored.

The controller 80 is connected, for example, via a bus or the like, to alignment mechanisms 5a, 5b, the monitor 8, the light source 41, the driver 48d, the driver 61a, the light reception element 62, the light source 71, a storage apparatus 81, an operating unit 85, and the like.

The storage apparatus 81 is a re-writable, non-volatile storage apparatus. As the storage apparatus 81, it is possible to apply various storage apparatuses, such as a hard disk, a flash memory, and a USB memory. Also, in the storage apparatus 81, at least a program for causing the ophthalmic apparatus 1 to perform various operations, such as the photographing operation, may be stored, for example.

The ACA image captured by the ophthalmic apparatus 1 may be saved in the storage apparatus 81 or may be displayed on the monitor 8.

The operating unit 85 is an input interface of the ophthalmic apparatus 1. As the operating unit 85 is operated by the examiner, an instruction corresponding to the operation is input to the controller 80. The operating unit 85 may include a pointing apparatus such as a mouse and a touch panel, and a keyboard, for example. Also, in the ophthalmic apparatus 1, the joystick 7 which is operated for alignment may be utilized as part of the operating unit 85.

### <Description of operation>

Next, referring to Fig. 6 to Fig. 12, the operation of the ophthalmic apparatus 1 will be described. For convenience, in the following description, it is assumed that the photographing operation for the ACA image and the display/viewing of an ACA image that has been captured in advance are performed in a series of events. However, this is not a limitation, and the photographing operation and the display/viewing are not required to be performed in a series, and may be performed each independently.

### <Photographing operation>

First, the controller 80 turns on the light source 71, and starts projection of the fixation target (S1). This makes it possible to guide the line of sight of the examinee's eye E.

Then, alignment of the photographing optical system 30 with respect to the examinee's eye E is performed (S2). The alignment of the ophthalmic apparatus 1 is performed by manually or automatically adjusting the positional relationship between the examinee's eye and the photographing optical system 30 in accordance with the position of a feature in the ACA image captured by the ophthalmic apparatus 1, the position concerning the radial direction about the fixation optical axis L1.

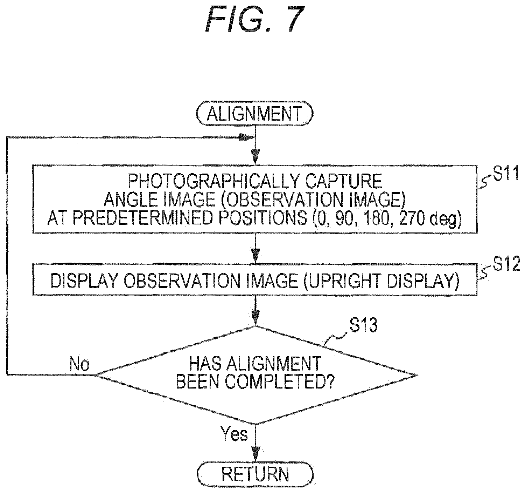

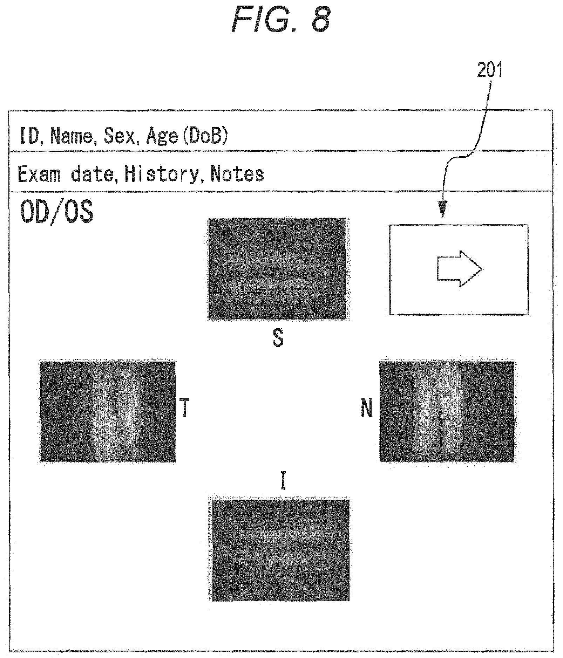

As an example, here the alignment is performed manually. Referring to Fig. 7 and Fig. 8, the manual alignment operation for the ophthalmic apparatus 1 will be described.

First, the examiner operates the joystick 7 and the like to cause the photographing optical system 30 to become closer to the examinee's eye E visually, so that the tip-end of the objective reflector 50 is at a distance of the order of several millimeters from the cornea of the examinee's eye E. At this time, in addition, the gel G is interposed between the examinee's eye E and the objective reflector 50.

For example, after the tip-end of the objective reflector 50 has been moved close to the cornea of the examinee's eye E, the operating unit 85 is operated, whereby the controller 80 starts the operation for acquiring and displaying observation images (S11, S12). As the observation images, ACA images captured in chronological order are utilized. In the Example, time-series still images obtained at regular time intervals are utilized as the observation images. However, this is not a limitation, and the observation images may be a moving image.

In the Example, the controller 80 switches the photographing position periodically between a plurality of photographing positions, and captures the ACA image at each photographing position, as needed. As illustrated in Fig. 8, the controller 80 causes the ACA image at each photographing position to be displayed simultaneously on the monitor 8. At this time, each of the ACA images is switched in chronological order and displayed.

The photographing positions of the ACA images utilized as an observation image may be determined in advance. For example, for the alignment in one direction of the X-direction and the Y-direction, two ACA images of which the positional relationship of the photographing positions is in a diagonal relationship along that one direction on the entire circumference of the ACA (in other words, in a symmetric relationship with respect to the fixation optical axis L1) are utilized. In the Example, because the alignment concerning both the X-direction and the Y-direction is performed based on the ACA images, the ophthalmic apparatus 1 acquires the ACA images at the four photographing positions of 0°, 90°, 180°, and 270° on the entire circumference of the ACA, and displays the ACA images as the observation images.

Note that the controller 80 causes the light-decentering section 48 to be rotated from the position of 0° by 1/4 of the circumference at a time (that is, by 90° at a time), and causes the rotation to be stopped temporarily at every rotation by 1/4 of the circumference. Then, during the stop, the controller 80 causes the light projecting optical system 40 to irradiate illuminating light to capture an ACA image. In this way, the ACA image may be captured while the rotation is stopped. During the alignment, the controller 80 causes the light-decentering section 48 to be rotated repeatedly, and captures the ACA images.

The ACA images obtained at the four photographing positions of 0°, 90°, 180°, 270° are then displayed as the observation images on the monitor 8. At this time, the controller 80 selects upright display out of upright display and reverse display, and causes the ACA images to be displayed (S12). Also, the display positions of the four ACA images are set such that the positional relationship of the respective photographing positions as viewed from the photographing optical system 30 and the positional relationship of the respective ACA images on the monitor 8 correspond to each other in terms of top-bottom and right-left relationships.

On the basis of such observation images, the examiner learns the alignment state of the photographing optical system 30 relative to the examinee's eye E. In the case of upright display, the top-bottom and right-left of the examinee's eye E and the top-bottom and right-left of the ACA images on the monitor 8 correspond to each other. The positional relationship of the respective ACA images on the monitor 8 and the positional relationship of the respective photographing positions as viewed from the photographing optical system 30 also correspond to each other. Thus, it becomes easier to intuitively learn, from the ACA images on the monitor 8, the direction of alignment error concerning the X- and Y-directions (and the direction in which to cause the photographing optical system 30 to be moved in order to eliminate the alignment error). As a result, it becomes easier to smoothly perform the manual alignment adjustment.

When the observation images are displayed as described above, a position indicator may be displayed in association with each of the observation images. By displaying the position indicator, it becomes possible for the examiner to learn the direction of alignment error in a more satisfactory manner. As illustrated in Fig. 8, in the case of the right eye, the position indicators are shown by means of: the letter "N" for the image at the photographing position of 0° (that is, the image at the photographing position on the nose side); the letter "S" for the image at the photographing position of 90° (that is, the image at the upper photographing position); the letter "T" for the image at the photographing position of 180° (that is, the image at the photographing position on the temple side; and the letter "I" for the image at the photographing position of 270° (that is, the image at the lower photographing position). In this way, the direction of alignment error concerning the X- and Y-directions can be learned more reliably. Note that the letters "N", "S", "T", and "I" are respectively the capital letters of nasal, superior, temporal, and inferior.

Further, in the Example, in the case of the left eye, the position indicator shown with respect to the image at the photographing position of 0° and the position indicator shown with respect to the image at the photographing position of 180° are exchanged with respect to the case of the right eye. The controller 80 may acquire in advance right/left eye-identifying information associated with each ACA image and indicating whether the examinee's eye is the right eye or the left eye. On the basis of the right/left eye-identifying information, at least some of the position indicators may then be switched and displayed. The right/left eye-identifying information may be acquired based on an operation input made on the operating unit 85, for example. Also, if it is possible, by detecting the position of the moving base 4 relative to the base 3 when an ACA image is being captured, to identify whether the right eye or the left eye is captured, the right/left eye-identifying information may be acquired based on a position detection signal pertaining to the moving base 4.

When the observation images are being displayed as described above, the examiner can operate the joystick 7 and the like, and adjust the positional relationship between the examinee's eye E and the photographing optical system 30 as follows.

When two ACA images at two photographing positions in a diagonal relationship on the entire circumference of the ACA (in other words, a symmetric relationship with respect to the fixation optical axis L1) are captured/displayed, the examiner operates the joystick and the like so that the features in the two ACA images are present at the same position with respect to the radial direction.

For example, the alignment for the X-direction (horizontal direction) is performed based on the two ACA images at the positions of 0° and 180°, among the four ACA images at the photographing positions of 0°, 90°, 180°, and 270°. More specifically, the alignment adjustment is performed as the examiner operates the operating unit 85 and the like so that the positions of the trabecular meshwork (an example of feature) concerning the radial direction (in this case, horizontal direction) become substantially the same between the two ACA images at the positions of 0° and 180°. In this way, the positional relationship between the examinee's eye E and the photographing optical system 30 concerning the X-direction (horizontal direction) is adjusted.

Further, the controller 80 also performs the alignment for the Y-direction (top-bottom direction) based on the two ACA images at the positions of 90° and 270° among the four. More specifically, the alignment adjustment is performed as the examiner operates the operating unit 85 and the like so that the positions of the trabecular meshwork (an example of feature) concerning the radial direction (in this case, vertical direction) become substantially the same between the two ACA images at the positions of 90° and 270°. In this way, the positional relationship between the examinee's eye E and the photographing optical system 30 concerning the Y-direction (vertical direction) is adjusted.

Next, the alignment for the Z-direction will be described. For example, the shorter the actuation distance (the distance between the examinee's eye E and the objective reflector 50) is, the closer the trabecular meshwork (an example of feature) will be to the distal side with respect to the fixation optical axis L1 in the ACA image. On the other hand, the greater the actuation distance is, the closer the trabecular meshwork will be to the fixation optical axis L1 in the ACA image.

Accordingly, for example, the examiner confirms whether, in two ACA images at photographing positions which are symmetric with respect to the fixation optical axis LI, the trabecular meshwork is disposed in a target position determined in advance in the ACA images. In this case, preferably, the target position is set toward the middle in the ACA images. During the alignment adjustment, a graphic (reticle) indicating the target position may be electronically or optically superimposed with respect to the ACA images (observation images) on the monitor. For example, in Fig. 8, a rectangular graphic indicating the target position of the trabecular meshwork is superimposed as a reticle over each observation image.

If the confirmation indicates that the trabecular meshwork is closer to the fixation optical axis L1 than to the target position, alignment adjustment is performed in a direction to move the photographing optical system 30 closer to the examinee's eye E. On the other hand, if the trabecular meshwork is closer to the distal side with respect to the fixation optical axis L1 than to the target position, alignment adjustment is performed in a direction to move the photographing optical system 30 away from the examinee's eye E. Here, due to the ACA being formed in an elliptical shape, there may be cases where it is difficult to perform the adjustments so as to satisfy the target position in both the X-direction and the Y-direction. In this case, the positional relationship concerning the actuation distance direction may be adjusted such that the central position (for example, an average position) between the feature position concerning the X-direction and the feature position concerning the Y-direction is disposed at the target position.

In the Example, the controller 80 during manual alignment detects the trabecular meshwork (an example of feature) in each ACA image, and, based on the position of the detected feature, outputs guidance information 201. The guidance information 201 is information for guiding the operation of the operating unit during manual alignment. The guidance information guides the direction of movement of the optical unit 10 to a correct alignment position. While in Fig. 8 the guidance information 201 is an arrow graphic, this is not a limitation. For example, the guidance information 201 may be text information using letters such as "Up" and "Down", voice information, or other information.

The alignment adjustment for the X- and Y-directions and the alignment adjustment for the Z-direction described above may be alternately repeated a plurality of times. In the Example, if it is determined that a predetermined alignment completion condition has been satisfied based on the ACA images, or when an alignment completion signal has been input through an operation of the operating unit 85, the process concerning the alignment is discontinued (S13: Yes). If the predetermined alignment completion condition is not satisfied, the acquisition and display of the observation image is continued (S13: No).

As a result of the alignment operation, the center of the examinee's eye with respect to the entire circumference of the ACA and the optical axis of the objective reflector 50 are substantially aligned with each other. Further, when the light-decentering section 48 is rotated and the ACA image is captured at each photographing position on the entire circumference of the ACA, the trabecular meshwork that is the feature comes to be included in each ACA image in a satisfactory manner.

Referring back to Fig. 6 and continuing with the description, after the alignment has been completed, the photographing operation is performed (S3).

In the Example, as the result of the photographing operation, ACA images that have been focused with respect to the trabecular meshwork are acquired as captured images. For example, in the Example, ACA images are captured in succession at 16 photographing positions obtained by dividing the entire circumference of the ACA into 16 portions. The controller 80, after controlling the polarizer 48 and setting the photographing position at one of the 16 locations, captures a plurality of ACA images in mutually different focusing states while moving the focusing lens 61. The focusing lens 61 is moved in a predetermined range. The photographing operation is repeatedly performed by switching the photographing position.

The controller 80 selects, as the captured image for each photographing position, an image that is in focus most preferably with respect to the trabecular meshwork. The captured image that has been selected may be stored in the memory 82. Note that at this time, the captured image may be selected by comparing the contrast in an image region in the vicinity of the trabecular meshwork in each ACA image.

After the captured images have been obtained for all of the photographing positions, the photographing operation is discontinued. As the result of the photographing operation performed with respect to each photographing position, a plurality of (here, 16) ACA images that is in focus with respect to the trabecular meshwork at each photographing position can be obtained (as captured images).

The controller 80 may cause the ACA images that have been captured as described above to be displayed on the monitor 8 (S4).

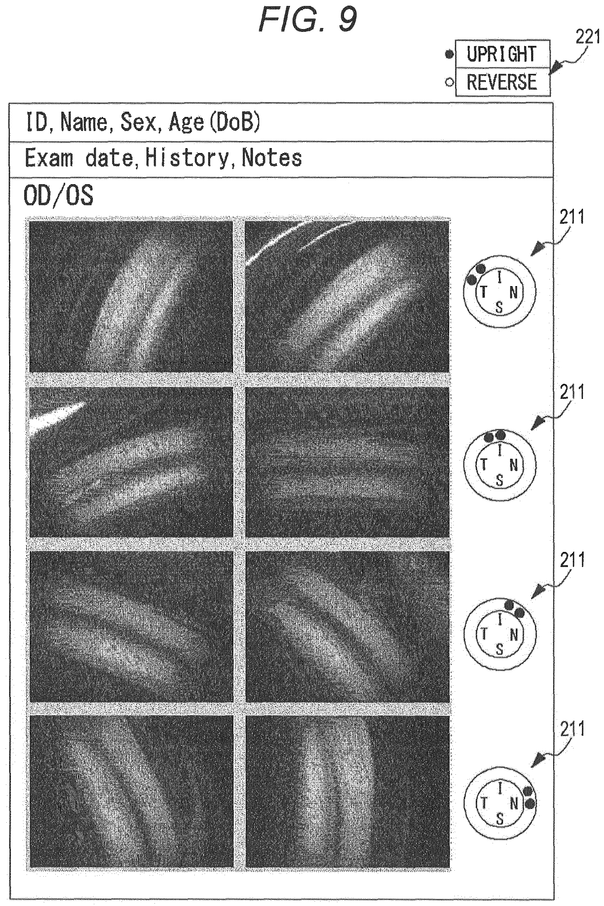

In the Example, the ophthalmic apparatus 1 can cause a plurality of ACA images with mutually different photographing positions to be displayed in three types of layout, i.e., first to third layouts, as illustrated in Fig. 9 to Fig. 12.

In the Example, a display method selection button 221 may be provided on the monitor 8 to select one of upright display and reverse display when the ACA images are displayed in the first to third layouts. If a selecting operation for "upright display" has been input via the button 221, the controller 80 causes each of the ACA images to be displayed in upright display. Also, if a selecting operation for "reverse display" has been input via the button 221, the controller 80 causes each of the ACA images to be displayed in reverse display. In the Example, in an initial state, upright display is selected. However, this is not a limitation. It may be made possible to set/save, based on a setting operation, setting data concerning whether upright display or reverse display is to be selected in the initial state, and the controller 80 may select the display method based on the setting data when the ACA images are displayed.

In the first layout, each of the ACA images is displayed on the monitor 8 as is (without being collaged). The ACA images having mutually different photographing positions are arranged vertically and horizontally and displayed on the monitor 8. As a concrete example, in Fig. 9, every two of eight ACA images are disposed side by side (that is, the eight ACAs images are arranged in two vertical columns).

Next to every two ACA images disposed side by side, a photographing position graphic 211 is then disposed. The photographing position graphic 211 is a type of photographing position indicator. The photographing position graphic 211 indicates the photographing position of each ACA image with respect to the entire circumference of the ACA.

The photographing position graphic 211 represents the entire circumference of the ACA by means of a ring-shaped figure. In the ring-shaped figure, an indicator (a dot in Fig. 9) is added to the position corresponding to the photographing position, whereby the photographing position is indicated. In the Example of Fig. 9, the photographing position for two ACA images is indicated by a single photographing position graphic 211. In this display mode, it is also possible for the examiner to easily learn, from the inclined state of the tissue in each of the ACA images, the correspondence between the two ACA images and the photographing position.

In the photographing position graphic 211, the orientation of the ring-shaped figure (more specifically, the top-bottom and right-left orientations) is indicated by the letters [T], [I], [N], and [S]. Also, in the Example, it is possible to identify, from the positional relationship of the letters (mainly [T] and [N]), whether the ACA image being displayed on the monitor is a right-eye image or a left-eye image. However, this is not a limitation, and information indicating whether the ACA image displayed on the monitor 8 is a right-eye image or a left-eye image may be displayed as information independent from the photographing position indicator. For example, in Fig. 9, one of OD (right eye)/OS (left eye) may be displayed selectively on the monitor 8 so as to indicate whether the ACA image on the monitor 8 is that of the right eye or the left eye.

In the second layout, an annular composite image composed of a plurality of ACA images is displayed. In the annular composite images shown in Fig. 10 and Fig. 11, 16 ACA images captured at the 16 photographing positions described above are collaged. In this way, it becomes possible for the examiner to easily confirm the state of the entire circumference of the ACA.

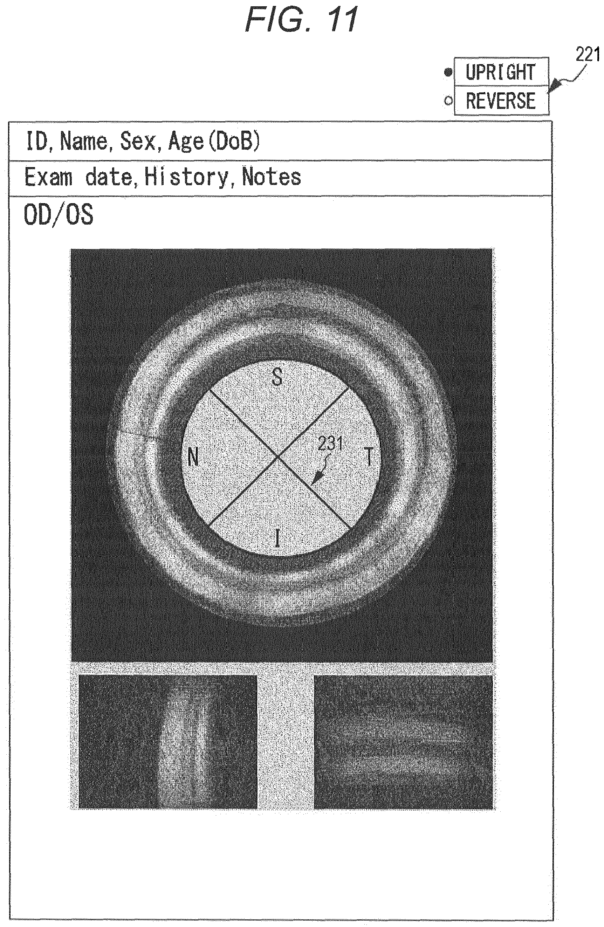

In Fig. 10 and Fig. 11, as a type of photographing position indicator, the letters [T], [I], [N], and [S] are displayed respectively in association with the top-bottom and right-left positions of the annular composite image. In this way, the examiner can easily learn the orientation of the annular composite image.

Further, a graphic 231 (see Fig. 11) is displayed for only one of the case where the annular composite image is upright-displayed and the case where the annular composite image is reverse-displayed. The graphic 231 identifies the display method that has been selected concerning the ACA images on the monitor 8, and is a type of identifying information. The graphic 231 is a cross sign, and is superimposed over the pupil portion of the annular composite image. For example, the cross sign is displayed when upright display has been selected out of upright display and reverse display.

Further, in the second layout, together with the annular composite image, some of the ACA images of which the annular composite image has been composed are displayed on the monitor 8. In the annular composite image, an indicator (here, rectangular frame lines) indicating the regions corresponding to the some of the ACA images is displayed in an associated manner as a position indicator for the some of the ACA images. In this way, the examiner can learn the correspondence between some of the ACA images and the annular composite image in a satisfactory manner.

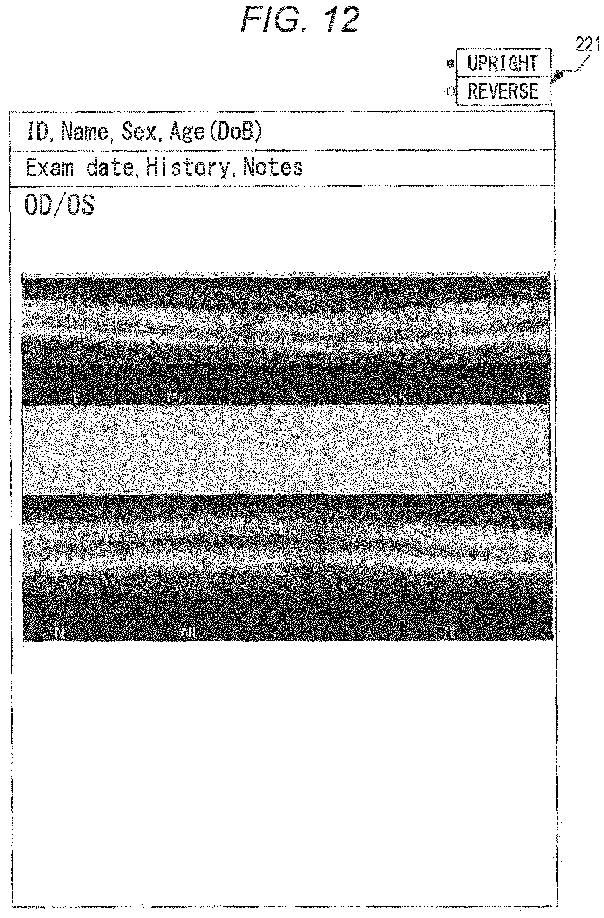

In the third layout, a linear composite image obtained by linearly collaging a plurality of ACA images is displayed. In Fig. 12, linear composite images for the entire circumference of the ACA are shown in two levels. However, this is not a limitation, and a linear composite image for the entire circumference of the ACA may be displayed in one level. In Fig. 12, as a type of photographing position indicator, the letters [T], [I], [N], [S], [TS], [NI], [NS], [NS], and the like are displayed in association with each region of the linear composite image. In this way, the examiner can learn the correspondence between each region of the linear composite image and each position on the entire circumference of the ACA in a satisfactory manner.

Of course, the ACA images at the respective photographing positions are not required to be displayed simultaneously, and the images of some of the photographing positions may be selectively displayed on the monitor.

While the present disclosure has been described with reference to the embodiment as above, the present disclosure is not limited to the embodiment and may be modified in various ways.

### <Display method selection window>

The ophthalmic apparatus 1 is able to select the ACA image display method at least between upright display and reverse display. The display method may be set in advance for each operation scene based on an operation of the operating unit by the examiner. In this way, the controller selects the display method set in advance in each operation scene, and causes the ACA images to be displayed by the selected display method.

It should be noted, however, that it may be made possible to change the display method from the one set in advance to another display method, as needed, by performing a predetermined operation during each operation scene.

### <Selection of display method during alignment>

Further, while the Example has been described where upright display is selected for the alignment, this is not a limitation. For example, even during an automatic or manual alignment, it may be made possible to select upright display or reverse display based on an operation performed by the examiner.

## Claims

1. An ophthalmic apparatus comprising:
an image data acquisition means which acquires image data of an ACA image of an examinee's eye captured by a photographing optical system which includes a light projecting optical system for projecting light to an ACA region of the examinee's eye and a light reception optical system having a light reception element for receiving reflected light from the ACA region; and
a control means which causes the ACA image to be displayed on a monitor based on the image data, wherein the control means selects a display method for the ACA image between upright display for displaying an upright image in which the top-bottom and right-left of the examinee's eye correspond to the top-bottom and right-left of the ACA image on the monitor, and reverse display for displaying a mirror image or an inverted image with respect to the upright image.

2. The ophthalmic apparatus according to claim 1, wherein
the photographing optical system is able to capture the ACA image at a plurality of photographing positions on an entire circumference of an ACA, and
the control means causes a plurality of ACA images with mutually different photographing positions to be displayed on the monitor simultaneously by one display method selected out of the upright display and the reverse display.

3. The ophthalmic apparatus according to claim 2, wherein the control means causes the plurality of ACA images with mutually different photographing positions to be displayed on the monitor at display positions which are respectively associated with the photographing positions and are annularly arranged on the monitor, by one display method selected out of the upright display and the reverse display.

4. The ophthalmic apparatus according to claim 3, wherein the control means generates an annular composite image by annularly arranging and collaging the ACA images captured at the plurality of photographing positions on the entire circumference of the ACA, and causes the annular composite image to be displayed on the monitor by one display method selected out of the upright display and the reverse display.

5. The ophthalmic apparatus according to claim 3 or 4, wherein the control means, when the upright display has been selected, causes the correspondence between the display positions and the respective photographing positions of the examinee's eye to be aligned in top-bottom and right-left terms, and, when the reverse display has been selected, causes the correspondence between the respective display positions and the respective photographing positions of the examinee's eye to be reversed from the case of the upright display.

6. The ophthalmic apparatus according to any one of claims 1 to 5, wherein the control means causes the monitor to display, together with the ACA image, identifying information for identifying the display method selected with regard to the ACA image.

7. The ophthalmic apparatus according to claim 6, wherein the control means causes information indicating the photographing position of the ACA image to be displayed as the identifying information.

8. The ophthalmic apparatus according to claim 6, wherein the control means, when the upright display has been selected, causes a cross sign to be displayed as the identifying information in a region surrounded by a plurality of ACA images on the monitor.

9. The ophthalmic apparatus according to any one of claims 1 to 8, further comprising a moving mechanism which, for alignment adjustment of the photographing optical system relative to the examinee's eye, causes the photographing optical system to be moved relative to the examinee's eye in at least one of a top-bottom direction and a right-left direction, based on an operation performed by an examiner with respect to an operating unit, wherein
the control means further controls a photographing operation for the ACA image performed by the photographing optical system, and, in order to cause the alignment adjustment to be performed, causes the ACA image to be captured successively, and causes the ACA image to be displayed by the upright display, as needed.

10. The ophthalmic apparatus according to any one of claims 1 to 9, wherein the control means:
receives an operation performed by an examiner with respect to an operating unit; and
performs the selection of the upright display or the reverse display based on the operation.

11. An ophthalmic apparatus comprising:
an image data acquisition means which acquires image data of an ACA image of an examinee's eye captured by a photographing optical system which includes a light projecting optical system for projecting light to an ACA region of the examinee's eye and a light reception optical system having a light reception element for receiving reflected light from the ACA region; and
a control means which causes the ACA image to be displayed on a monitor based on the image data, and outputs, on the monitor, a position indicator indicating a position on the ACA region in association with all or a part of the ACA image.

12. The ophthalmic apparatus according to claim 11, wherein the control means outputs, as the position indicator, information identifying an orientation of the ACA region that is depicted in the ACA image.

13. The ophthalmic apparatus according to claim 11 or 12, wherein
the image data acquisition means acquires, together with image data of the ACA image, photographing position data indicating a photographing position of the ACA image, and
the control means outputs the position indicator based on the photographing position data corresponding to the ACA image displayed on the monitor.

14. The ophthalmic apparatus according to claim 13, wherein
the photographing optical system is able to switch the photographing position on an entire circumference of an ACA based on driving of a photographing position switch, and
the photographing position data is data indicating a state of the photographing position switch when the ACA image is being captured.

15. The ophthalmic apparatus according to any one of claims 11 to 13, wherein the control means causes the monitor to display a plurality of the ACA images with mutually different photographing positions simultaneously, and causes the position indicator to be displayed in association with some or all of the plurality of the ACA images being displayed on the monitor.

16. The ophthalmic apparatus according to claim 15, wherein the control means, when the plurality of ACA images is simultaneously displayed, sets the display position of each of the ACA images on the monitor in accordance with the photographing position of each of the ACA images on the ACA region.

17. The ophthalmic apparatus according to claim 15 or 16, wherein the control means:
obtains a composite image by collaging the plurality of ACA images with mutually different photographing positions, and causes the plurality of ACA images to be displayed on the monitor as the composite image; and
causes a position indicator indicating the photographing position of one or more of image regions of the composite image to be displayed in association with the one or more image regions.

18. The ophthalmic apparatus according to claim 17, wherein the control means causes a first ACA image which is the ACA image before being composed and the composite image obtained by the collaging including the first ACA image to be displayed simultaneously or in a switched manner, and causes the position indicator to be displayed commonly in association with each of the first ACA image and an image region in the composite image that corresponds to the first ACA image.

19. The ophthalmic apparatus according to claim 17, wherein the control means:
causes a first ACA image which is the ACA image before being composed and the composite image obtained by the collaging including the first ACA image to be displayed simultaneously or in a switched manner; and
causes an indicator highlighting an image region of the composite image corresponding to the first ACA image to be displayed as a position indicator of the first ACA image.

20. The ophthalmic apparatus according to any one of claims 11 to 17, wherein the control means:
causes an entire-circumference image, which is the ACA image representing an entire circumference of an ACA of the examinee's eye, and a partial image, which is the ACA image representing a partial region of the entire circumference of the ACA, to be displayed simultaneously or in a switched manner; and
causes the position indicator to be displayed commonly in association with each of the partial image and an image region of the entire-circumference image corresponding to the partial image.

21. The ophthalmic apparatus according to any one of claims 11 to 17, wherein the control means:
causes an entire-circumference image, which is the ACA image representing an entire circumference of an ACA of the examinee's eye, and a partial image, which is the ACA image representing a partial region of the entire circumference of the ACA, to be displayed simultaneously or in a switched manner; and
causes an indicator highlighting an image region of the entire-circumference image corresponding to the partial image to be displayed as a position indicator of the partial image.

22. The ophthalmic apparatus according to claim 16, wherein the control means:
selects a first display method by which the correspondence between the display position of each of the ACA images displayed simultaneously on the monitor and each of the photographing positions on the examinee's eye are aligned in top-bottom and right-left terms, or a second display method by which the correspondence between the display position of each of the ACA images simultaneously displayed on the monitor and each of the photographing positions on the examinee's eye is reversed from the case of the upright display; and
further switches the display position of the position indicator on the monitor in accordance with the selected display method.

23. The ophthalmic apparatus according to claim 22, wherein the control means, when the first display method has been selected, causes each of the ACA images to be displayed as an upright image in which the top-bottom and right-left of the examinee's eye and the top-bottom and right-left of the ACA image on the monitor correspond to each other, and, when the first display method has been selected, causes each of the ACA images to be displayed by a mirror image or an inverted image with respect to the upright image.
